# EUROPEAN PATENT APPLICATION

(11) **EP 4 484 418 A1**
(43) Date of publication of application: **01.01.2025**
(21) Application number: 23760413.7
(22) Date of filing: 24.02.2023
(51) Int. Cl.: C07D 401/04, A61K 31/454, A61P 29/00, A61P 17/06, A61P 19/02, A61P 1/00, A61P 25/00, A61P 35/00, A61P 43/00

(54) **USE OF NOVEL POMALIDOMIDE DERIVATIVE FOR TREATING BRAIN DISEASE**

(30) Priority: 25.02.2022 KR 20220024800; 24.06.2022 KR 20220077699
(71) Applicant: Aevis Bio, Inc., Daejeon 34141 (KR)
(72) Inventor: KIM, Dong Seok, Daejeon 34033 (KR); HWANG, Inho, Cheonan-si, Chungcheongnam-do 31145 (KR); KIM, Sun, Daejeon 34118 (KR)
(74) Representative: Maiwald GmbH
(86) International application number: PCT/KR2023/002639
(87) International publication number: WO 2023/163539

(57) **Abstract**

The present invention relates to a use of a novel pomalidomide derivative which is for treating brain diseases, and exhibits improved pharmacological effects as compared to pomalidomide. The novel pomalidomide derivative according to the present invention exhibits excellent binding to cereblon, has almost no cytotoxicity, is unlikely to cause teratogenic adverse effects, can control TNF-α expression, exhibits excellent *in vivo* pharmacokinetic stability while suppressing oxidative stress, ameliorates a decrease in motor function which is a symptom of Parkinson's disease, and increases tyrosine hydroxylase expression which is reduced by Parkinson's disease, and thus can be used for preventing or treating brain diseases such as Parkinson's disease.

## Description

### [Technical Field]

The present invention relates to a used of a novel pomalidomide derivative, which exhibits an improved pharmacological effect compared to pomalidomide, for treating brain diseases.

### [Background Art]

Thalidomide was a drug sold from the late 1950s to the 1960s to prevent morning sickness in pregnant women, but as teratogenic side effects were reported, its use and handling were prohibited for pregnant women as well as people of childbearing age or those who may become pregnant. However, interest in the drug has increased again after thalidomide was found to be clinically effective in the treatment of erythema nodosum leprosum (ENL), and treatments of HIV wasting syndrome and various types of cancer.

From the mechanism study on ENL activity, the anti-tumor necrosis factor-alpha (anti-TNF-α) action was confirmed, and specifically, thalidomide increases the degradation of TNF-α RNA and reduces its synthesis and secretion. Additional studies have shown that it is known as a co-stimulator of both CD8+ and CD4+ T cells, and it can also act as an angiogenesis inhibitor, as an inhibitor of the basic fibroblast growth factor (bFGF), the vascular endothelial growth factor (VEGF), and the transcription factor (NF-κB).

TNF-α and its family members play a pivotal role in a variety of physiological and pathological processes, including cell proliferation and differentiation, apoptosis, regulation of immune responses, and inflammation induction. TNF-α acts through two receptors such as TNFR1 and TNFR2. The former is expressed in all tissues and is the primary signaling receptor for TNF-α, and the latter is expressed primarily in immune cells and mediates more limited biological responses. When cells are exposed to TNF-α, the caspase cascade may be activated to kill the cells through apoptosis. In fact, the major cell surface molecules that can initiate apoptosis are ligands and receptors for TNF family members. For example, each death-inducing member of the TNF receptor family contains a cytoplasmic 'death domain' (DD), which is a protein-protein interaction motif significant for binding to a downstream component of the signaling pathway.

Recently, TRAIL, which is a tumor necrosis factor-related apoptosis-inducing ligand, has been shown to selectively induce apoptosis in tumor cells but not in most normal cells. TRAIL is known to mediate the apoptosis of thymocytes and play an important role in the induction of autoimmune diseases. However, more frequently, TNF-α receptor binding induces the activation of transcription factors such as AP-1 and NF-κB, which in turn induces genes involved in acute and chronic inflammatory responses. Therefore, the overproduction of TNF-α has been implicated in many inflammatory diseases such as rheumatoid arthritis, graft-versus-host disease, and Crohn's disease, and is known to further exacerbate ENL, septic shock, AIDS, dementia associated with Alzheimer's disease (AD).

Commercially available thalidomide-based treatments include thalidomide, bortezomib, lenalidomide, and pomalidomide, and the injectable drug, bortezomib, is steadily maintaining its market share and growing, and as an oral agent, lenalidomide is replacing the position of existing thalidomide, and is recording high growth in the market. Lenalidomide is a next-generation drug of thalidomide and shows better therapeutic effects than thalidomide through more powerful killing of cancer cells and immunoregulation. In cases of recurrence or refractoriness to existing treatment, in treatment with a combination of lenalidomide and dexamethasone, it is known that a disease-free survival period is 13.4 months, and an overall survival period is 38 months, which is known to be quite effective. As for side effects, a problem such as peripheral neuropathy that occurred with existing thalidomide has almost disappeared, and the side effect of bone marrow suppression has been slightly more severe, but it is known that there is no major problem when administering leukocyte stimulating factors. Pomalidomide, approved by the US FDA in 2013 as a therapeutic agent for recurrent and refractory multiple myeloma, is used when the disease progresses within 60 days after the final treatment in people who have previously been treated with at least two therapeutic agents, including lenalidomide and bortezomib. Pomalidomide directly inhibits angiogenesis and myeloma cell growth, and this dual effect is central to the activity of pomalidomide in myeloma rather than to other pathways such as TNF-α inhibition. Through not only the enhancement of IFN-γ, IL-2 and IL-10 expression but also the suppression of IL-6 expression, pomalidomide exhibits anti-angiogenic and anti-myeloma activities.

In US Patent No. 9,623,020, it has been confirmed that thio compounds were newly synthesized from thalidomide derivatives, and these thio compounds can regulate TNF-α activity and change the expression of immune cytokines by TCR-stimulated T cells.

As a result of intensive efforts to develop additional derivatives for improving the pharmaceutical effect of thalidomide, the present inventors confirmed that the compound of Chemical Formula 1 can exhibit the pharmaceutical effect the same as or higher than that of the conventional thalidomide derivative and have significantly improved pharmacodynamic stability *in vivo,* and exhibit an excellent therapeutic effect on brain diseases, particularly, Parkinson's disease. Thus, the present invention was completed.

### [Disclosure]

### [Technical Problem]

The present invention is directed to providing a use of a novel thalidomide derivative exhibiting an improved pharmacological effect compared to conventionally known thalidomide-based drugs for preventing or treating brain diseases.

### [Technical Solution]

To achieve the above purpose, the present invention provides a pharmaceutical composition for preventing or treating brain diseases, which includes a compound of Chemical Formula 1 or a pharmaceutically acceptable salt thereof as an active ingredient:

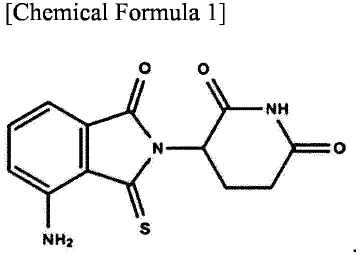

In the present invention, the brain disease may be a brain disease caused by oxidative stress.

In the present invention, the brain disease may be selected from the group consisting of a traumatic brain injury, Alzheimer's disease, Parkinson's disease, Huntington's disease, Lou Gehrig's disease, multiple system atrophy, Alzheimer's dementia, vascular dementia, frontotemporal dementia, Lewy dementia, ischemic stroke, hemorrhagic stroke, and multiple sclerosis.

In the present invention, the compound of Chemical Formula 1 may be non-teratogenic.

In the present invention, the pharmaceutical composition may be orally or parenterally administered.

In the present invention, the compound of Chemical Formula 1 or a pharmaceutically acceptable salt thereof may
(i) improve motor ability; and/or
(ii) increase the expression of tyrosine hydroxylase (TH) in the striatum and/or substantia nigra of the brain.

The present invention provides a food composition containing the compound of Chemical Formula 1:

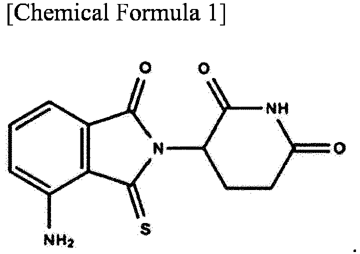

In the present invention, the food may be a health functional food for (i) reducing oxidative stress; (ii) improving motor ability; and/or (iii) improving tyrosine hydroxylase deficiency.

In the present invention, the food may be a health functional food for aiding the treatment of a brain disease.

The present invention provides a method of preventing or treating a brain disease, which comprises administering a compound of Chemical Formula 1 or a pharmaceutically acceptable salt thereof, or a composition comprising the same as an active ingredient into a subject in need thereof.

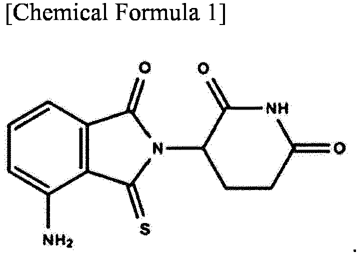

The present invention provides a compound of Chemical Formula 1 or a pharmaceutically acceptable salt thereof for use in preventing or treating a brain disease:

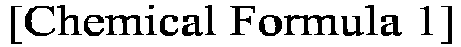

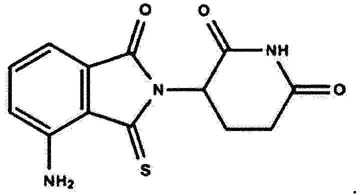

The present invention also provides a use of a compound of Chemical Formula 1 or a pharmaceutically acceptable salt thereof in the manufacture of a medicine for preventing or treating a brain disease:

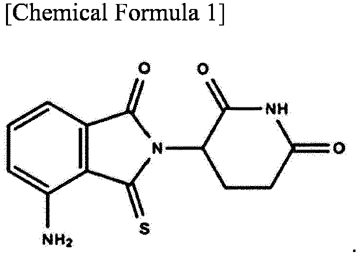

### [Advantageous Effects]

A novel pomalidomide derivative developed in the present invention can not only have very excellent binding strength to cereblon, almost no cytotoxicity, low possibility of side effects, teratogenicity, and regulate the expression of TNF-α and proinflammatory cytokines, but can also exhibit excellent effects of suppressing oxidative stress, having excellent pharmacodynamic safety *in vivo,* improving decreased motor skills, which are a sign of Parkinson's disease, and increasing the expression of tyrosine hydroxylase decreased in Parkinson's disease, and thus can be used for prevention or treatment of brain diseases such as Parkinson's disease.

### [Description of Drawings]

FIG. 1 shows the results of comparing the cereblon binding activity of 3-MP with pomalidomide through a competitive inhibition response *in vitro.*
FIG. 2 shows the results of evaluating the cytotoxicity of pomalidomide and 3-MP on the MM.1S cell line.
FIG. 3A shows the results of comparing intracellular Ikaros and Aiolos degradation effects by immunoblotting after treating MM.1S cells with each of pomalidomide and 3-MP.
FIG. 3B shows the results of comparing time-dependent degradation effects of Ikaros and Aiolos in cells after treating MM.1S cells with each of pomalidomide and 3-MP.
FIG. 4 shows the results of comparing the degree of intracellular SALL4 degradation by immunoblotting after treating Tera-1 cells with each of pomalidomide and 3-MP.
FIG. 5A shows the results of treating RAW264.7 cells with each of pomalidomide and 3-MP, and comparing an oxidative stress inhibition effect on iNOS and COX-2 through immune blotting.
FIG. 5B shows the results of treating BV-2 cells with each of pomalidomide and 3-MP, and comparing an oxidative stress inhibition effect by measuring a nitrite content in a cell culture solution.
FIG. 5C shows the results of comparing oxidative stress suppression effects on iNOS and COX-2 by immunoblotting after treating BV-2 cells with each of pomalidomide and 3-MP.
FIG. 5D shows the results of comparing oxidative stress suppression effects on iNOS and COX-2 at the mRNA level through RT-PCR after treating BV-2 cells with each of pomalidomide and 3-MP.
FIG. 6A shows the calibration curve for the plasma stability analysis of 3-MP. The linear equation and correlation coefficient (r) of the calibration curve are displayed on the graph. The Y-axis represents the peak area of the standard material, and the X axis represents the concentration (µg/mL).
FIG. 6B shows the results of analyzing the safety of 3-MP in human plasma over time.
FIG. 7A is the result of confirming a motor ability improvement effect caused by 3-MP administration in a mouse model with Parkinson's disease induced by MPTP administration through a pillar test.
FIG. 7B is the result of confirming a motor ability improvement effect caused by 3-MP administration in a mouse model with Parkinson's disease induced by MPTP administration through a grip strength test.
FIG. 7C is the result of confirming the increased expression of tyrosine hydroxylase (TH) in the striatum and/or substantia nigra of the brain, caused by 3-MP administration in a mouse model with Parkinson's disease induced by MPTP administration.

### [Modes of the Invention]

Unless defined otherwise, all technical and scientific terms used herein have the same meanings as commonly understood by one of ordinary skill in the art to which the present invention belongs. Generally, the nomenclature used herein and the experimental methods to be described below are well known in the art and commonly used.

In the present invention, a thio compound, which was an improved form of pomalidomide, was prepared, and the pharmaceutical efficacy of the novel compound was confirmed.

Specifically, the novel compound according to the present invention was identified to have superior cereblon binding activity compared to its parent compound, pomalidomide, and to have low cytotoxicity, indicating excellent *in vivo* safety. In addition, it was seen that the novel compound according to the present invention does not induce the degradation of the cereblon substrates, Aiolos and Ikaros, and SALL4, as strongly as pomalidomide, and thus the major side effects caused by thalidomide-based compounds can be reduced.

Meanwhile, it was confirmed that the novel compound according to the present invention can not only greatly improve motor ability in a Parkinson's disease animal model, but also increase the expression of tyrosine hydroxylase, which is a key factor of regulating dopamine synthesis.

In addition, the novel compound according to the present invention was able to inhibit the expression of various pro-inflammatory cytokines (TNF-α, IL-1β, IL-6, and IL-8), inhibit oxidative stress, and maintain *in vivo* safety for a long time via various administration routes, such as intravenous administration, oral administration, and intraperitoneal administration.

Therefore, one aspect of the present invention provides a pharmaceutical composition for preventing or treating a brain disease, which contains a compound of Chemical Formula 1 or a pharmaceutically acceptable salt thereof as an active ingredient:

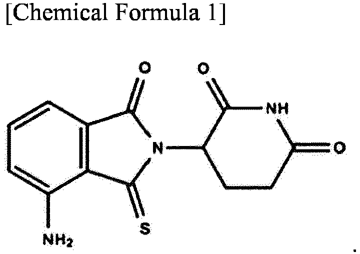

In the present invention, the compound of Chemical Formula 1 may be named 3-thiopomalidomide, which may be represented by C₁₃H₁₃N₃O₃S and has a molecular weight of 289.31. In the present invention, the compound of Chemical Formula 1 may be abbreviated as 3-MP.

In the present invention, the brain disease may be a brain disease caused by oxidative stress.

In the present invention, the brain disease may be one or more selected from the group consisting of a traumatic brain injury, Alzheimer's disease, Parkinson's disease, Huntington's disease, Lou Gehrig's disease, multiple system atrophy, Alzheimer's dementia, vascular dementia, frontotemporal dementia, Lewy dementia, ischemic stroke, hemorrhagic stroke, and multiple sclerosis, but the present invention is not limited thereto.

In the present invention, the compound of Chemical Formula 1 may be non-teratogenic.

In the present invention, the pharmaceutical composition may be orally or parenterally administered.

In the present invention, the compound of Chemical Formula 1 or a pharmaceutically acceptable salt thereof may
(i) improve motor ability; and/or
(ii) increase the expression of tyrosine hydroxylase (TH) in the striatum and/or substantia nigra of the brain.

In another aspect, the present invention provides a method of preventing or treating a brain disease, which includes administering a compound of Chemical Formula 1 or a pharmaceutically acceptable salt thereof, or a composition containing the same as an active ingredient into a subject in need thereof:

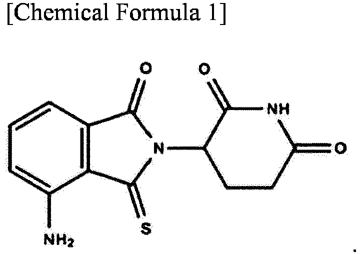

In still another aspect, the present invention provides a used of a compound of Chemical Formula 1 or a pharmaceutically acceptable salt thereof to prevent or treat a brain disease:

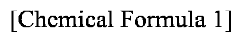

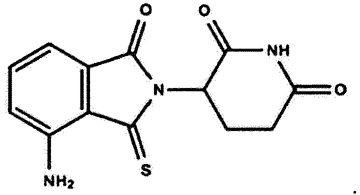

In yet another aspect, the present invention provides a use of a compound of Chemical Formula 1 or a pharmaceutically acceptable salt thereof to prepare a drug for preventing or treating a brain disease:

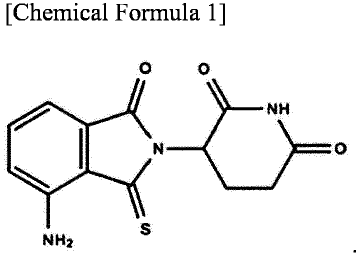

In the present invention, the pharmaceutical composition may be provided in a pharmaceutical dosage form, and the term "pharmaceutical composition" or "pharmaceutical formulation" used herein refers to a mixture containing a pharmaceutically acceptable excipient such as a diluent or carrier that renders a novel compound of the present invention particularly suitable for *in vivo* or *ex vivo* diagnostic or therapeutic use. According to some embodiments, a pharmaceutical composition that includes the composition of the present invention may be provided by administering it to a subject as needed. In some embodiments, the composition of the present invention may be administered to humans.

The "effective amount" or "therapeutically effective amount" used in the present invention refers to an amount of a compound or composition (e.g., a compound or composition of the present invention), which is sufficient to achieve a beneficial or desired result. An effective amount may be provided in one or more administrations, applications, or doses, and is not intended to be limited to a particular formulation or administration route.

The "pharmaceutically acceptable salt" used herein may be an acid forming a non-toxic acid addition salt containing a pharmaceutically acceptable anion, formed by, for example, an inorganic acid such as sulfuric acid, hydrochloric acid, nitric acid, phosphoric acid, hydrobromic acid, or hydroiodic acid, an organic acid such as tartaric acid, formic acid, citric acid, acetic acid, trifluoroacetic acid, gluconic acid, benzoic acid, lactic acid, fumaric acid, malonic acid, malic acid, salicylic acid, succinic acid, oxalic acid, propionic acid, aspartic acid, or glutamic acid, or a sulfonic acid such as methanesulfonic acid, ethanesulfonic acid, benzenesulfonic acid, p-toluenesulfonic acid, or naphthalenesulfonic acid. The compound represented by Chemical Formula 1 according to the present invention, which includes a free carboxyl substituent, may be the acid addition salt, and a sodium, calcium, or ammonium salt, or a pharmaceutically acceptable base addition salt, for example, an alkali metal or alkaline earth metal salt formed of lithium, sodium, potassium, calcium, or magnesium, an amino acid salt such as lysine, arginine, or guanidine, or an organic salt such as dicyclohexylamine, N-methyl-D-glucamine, tris (hydroxymethyl)methylamine, diethanolamine, choline, or triethyl amine.

The compound of Chemical Formula 1 according to the present invention may be converted to its salt by a conventional method, and the preparation of the salt may be easily performed by one of ordinary skill in the art based on the structure Chemical Formula 1 without any separate explanation.

The description of the pharmaceutical composition provided herein principally relates to a pharmaceutical composition for administration to humans, but those of ordinary skill in the art will understand that such a composition is generally suitable for administration to all kinds of animals. That is, the pharmaceutical composition according to the present invention may also be administered to animals requiring veterinary care, for example, other mammals such as domestic animals (e.g., dogs, cats, etc.), farm animals (e.g., cattle, sheep, pigs, horses, etc.), and laboratory animals (e.g., rats, mice, guinea pigs, etc.). Experienced veterinary pharmacologists with a good understanding of the modifications of the pharmaceutical composition for administration to various animals can design and/or execute such modifications simply by routine experimentation if needed.

The pharmaceutical composition described herein may be prepared by any method known in the field of pharmacology or as discussed later in the text. Generally, such a preparation method includes mixing the active ingredient with an excipient and/or one or more other auxiliary ingredients, and if needed or desired, forming and/or packaging the resulting product into desired single- or multi-dose units.

The pharmaceutical composition of the present invention may be manufactured, packaged, and/or sold unpackaged in a single unit dose and/or a plurality of single unit doses. As used in the present invention, "unit dose" is an individual amount of pharmaceutical composition including a predetermined amount of active ingredient. The amount of active ingredient is generally equal to the dose of active ingredient administered to a subject and/or a convenient fraction of such a dose, for example, 1/2 or 1/3 of the dose.

The relative amounts of the active ingredient, pharmaceutically acceptable excipients, and/or any additional ingredient in the pharmaceutical composition of the present invention will vary depending on the identity, size, and/or disorder of a subject to be treated, and an administration route of the composition. As an example, the composition may include 0.001 to 100 %(w/w) of active ingredient.

As used herein, the pharmaceutically acceptable excipients include all solvents, dispersion media, diluents, or other liquid vehicles, dispersions, suspension aids, surfactants, isotonic agents, thickeners, or emulsifiers, preservatives, solid binders, and lubricants, which are suitable for a particular dosage form. Remington's literature [The Science and Practice of Pharmacy, 21st Edition, A. R. Gennaro, (Lippincott, Williams & Wilkins, Baltimore, MD, 2006] discloses various excipients used in the preparation of pharmaceutical compositions and known techniques for their preparation. Their use is considered to be within the scope of the present invention except for any conventional carrier or medium that cannot coexist with a material or derivative thereof by imparting an undesirable biological effect or interacting with other components of the pharmaceutical composition in a harmful manner. The pharmaceutically acceptable excipients are at least 95%, 96%, 97%, 98%, 99%, or 100% pure.

The excipients are approved for human and veterinary use. In some embodiments, the excipients are approved by the US FDA. In some embodiments, the excipients are pharmaceutical grade. In some embodiments, the excipients meet the standards of the United States Pharmacopoeia (USP), European Pharmacopoeia (EP), British Pharmacopoeia, and/or International Pharmacopoeia (EP).

The pharmaceutically acceptable excipients used in the preparation of the pharmaceutical composition may include inert diluents, dispersants, and/or granulating agents, surfactants and/or emulsifiers, disintegrants, binders, preservatives, buffers, lubricants, and/or oils, but the present invention is not limited thereto.

These excipients may be included in the preparations of the present invention. Excipients such as cocoa butter and suppository waxes, colorants, coating agents, sweeteners, flavoring agents, and perfuming agents may be included in the composition at the formulator's discretion.

Exemplary diluents include calcium carbonate, sodium carbonate, calcium phosphate, dicalcium phosphate, calcium sulfate, calcium hydrogen phosphate, sodium lactose phosphate, sucrose, cellulose, microcrystalline cellulose, kaolin, mannitol, sorbitol, inositol, sodium chloride, dry starch, corn starch, powdered sugar, and a combination thereof, but the present invention is not limited thereto.

Exemplary granulating agents and/or dispersing agents include potato starch, corn starch, tapioca starch, sodium starch glycolate, clay, alginic acid, guar gum, citrus pulp, agar, bentonite, cellulose and wood products, natural sponges, cation-exchange resins, calcium carbonate, silicate, sodium carbonate, cross-linked poly(vinyl-pyrrolidone) (crospovidone), sodium carboxymethyl starch (sodium starch glycolate), carboxymethyl cellulose, cross-linked sodium carboxymethyl cellulose (croscarmellose), methylcellulose, pregelatinized starch (Starch 1500), microcrystalline starch, water-insoluble starch, calcium carboxymethyl cellulose, magnesium aluminum silicate (Veegum), sodium lauryl sulfate, quaternary ammonium compounds, and a combination thereof, but the present invention is not limited thereto.

Exemplary surfactants and/or emulsifiers include natural emulsifiers (e.g., acacia, agar, alginic acid, sodium alginate, tragacanth, chondrux, cholesterol, xanthan, pectin, gelatin, egg yolk, casein, wool fat, wax, and lecithin), colloidal clays (e.g., bentonite [aluminum silicate] and Veegum [magnesium aluminum silicate]), long-chain amino acid derivatives, high molecular weight alcohols (e.g., stearyl alcohol, cetyl alcohol, oleyl alcohol, triacetin monostearate, ethylene glycol distearate, glyceryl monostearate, and propylene glycol monostearate, and polyvinyl alcohol), carbomers (e.g., carboxy polymethylene, polyacrylic acid, acrylic acid polymers, and carboxyvinyl polymers), carrageenan, cellulose derivatives (e.g., carboxymethyl cellulose sodium, powdered cellulose, hydroxymethyl cellulose, hydroxypropyl cellulose, hydroxypropyl methylcellulose, and methyl cellulose), sorbitan fatty acid esters (e.g., polyoxyethylene sorbitan monolaurate [Tween 20], polyoxyethylene sorbitan [Tween 60], polyoxyethylene sorbitan monooleate [Tween 80], sorbitan monopalmitate [Span 40], sorbitan monostearate [Span 60], sorbitan tristearate [Span 65], glyceryl monooleate, sorbitan monooleate [Span 80]), polyoxyethylene esters (e.g., polyoxyethylene monostearate [Myrj 45], polyoxyethylene hydrogenated castor oil, polyethoxylated castor oil, polyoxymethylene stearate, and Solutol), sucrose fatty acid esters, polyethylene glycol fatty acid esters (e.g., Cremophor), polyoxyethylene ethers (e.g., polyoxyethylene lauryl ether [Brij 30]), poly(vinyl-pyrrolidone), diethylene glycol monolaurate, triethanolamine oleate, sodium oleate, potassium oleate, ethyl oleate, oleic acid, ethyl laurate, sodium lauryl sulfate, Pluronic F 68, Poloxamer 188, cetrimonium bromide, cetyl pyridinium chloride, benzalkonium chloride, docusate sodium, and/or a combination thereof, but the present invention is not limited thereto.

Exemplary binders include starches (e.g. corn starch and starch paste); gelatin; sugars (e.g. sucrose, glucose, dextrose, dextrin, molasses, lactose, lactitol, and mannitol); natural and synthetic gums (e.g. acacia, sodium alginate, Irish moss extract, panwar gum, ghatti gum, mucilage of isapgol husks, carboxymethylcellulose, methylcellulose, ethylcellulose, hydroxyethylcellulose, hydroxypropylcellulose, hydroxypropyl methylcellulose, microcrystalline cellulose, cellulose acetate, poly(vinyl-pyrrolidone), magnesium aluminum silicate (Veegum), and larch arabinogalactan); alginates; polyethylene oxides; polyethylene glycols; inorganic calcium salts; silicic acid; polymethacrylates; waxes; water; alcohols; and a combination thereof, but the present invention is not limited thereto.

Exemplary preservatives may include antioxidants, chelating agents, antimicrobial preservatives, antifungal preservatives, alcohol preservatives, acidic preservatives, and other preservatives. Exemplary antioxidants include alpha tocopherol, ascorbic acid, ascorbyl palmitate, butylated hydroxyanisole, butylated hydroxytoluene, monothioglycerol, potassium metabisulfite, propionic acid, propyl gallate, sodium ascorbate, sodium bisulfite, sodium metabisulfite, and sodium sulfite, but the present invention is not limited thereto. Exemplary chelating agents include ethylenediaminetetraacetic acid (EDTA), citric acid monohydrate, disodium edetate, dipotassium edetate, edetic acid, fumaric acid, malic acid, phosphoric acid, sodium edetate, tartaric acid, and trisodium edetate. Exemplary antimicrobial preservatives include benzalkonium chloride, benzethonium chloride, benzyl alcohol, bronopol, cetrimide, cetylpyridinium chloride, chlorhexidine, chlorobutanol, chlorocresol, chloroxylenol, cresol, ethyl alcohol, glycerin, hexetidine, imidurea, phenol, phenoxyethanol, phenylethyl alcohol, phenylmercuric nitrate, propylene glycol, and thimerosal, but the present invention is not limited thereto. Exemplary antifungal preservatives include butyl paraben, methyl paraben, ethyl paraben, propyl paraben, benzoic acid, hydroxybenzoic acid, potassium benzoate, potassium sorbate, sodium benzoate, sodium propionate, and sorbic acid, but the present invention is not limited thereto. Exemplary alcohol preservatives include ethanol, polyethylene glycol, phenol, phenol-based compounds, bisphenol, chlorobutanol, hydroxybenzoate, and phenylethyl alcohol, but the present invention is not limited thereto. Exemplary acidic preservatives include vitamin A, vitamin C, vitamin E, beta-carotene, citric acid, acetic acid, dehydroacetic acid, ascorbic acid, sorbic acid, and phytic acid, but the present invention is not limited thereto. Other preservatives include tocopherol, tocopherol acetate, deteroxime mesylate, cetrimide, butylated hydroxyanisole (BHA), butylated hydroxytoluende (BHT), ethylenediamine, sodium lauryl sulfate (SLS), sodium lauryl ether sulfate (SLES), sodium bisulfite, sodium metabisulfite, potassium sulfite, potassium metabisulfite, Glydant Plus, Phenonip, methyl paraben, Germall 115, Germaben II, Neolone, Kathon, and Euxyl, but the present invention is not limited thereto. In a specific embodiment, the preservative is an antioxidant. In another embodiment, the preservative is a chelating agent.

Exemplary buffers include citrate buffer solutions, acetate buffer solutions, phosphate buffer solutions, ammonium chloride, calcium carbonate, calcium chloride, calcium citrate, calcium glubionate, calcium gluceptate, calcium gluconate, D-gluconic acid, calcium glycerophosphate, calcium lactate, propanoic acid, calcium levulinate, pentanoic acid, dibasic calcium phosphate, phosphoric acid, tribasic calcium phosphate, calcium hydroxide phosphate, potassium acetate, potassium chloride, potassium gluconate, potassium mixtures, dibasic potassium phosphate, monobasic potassium phosphate, potassium phosphate mixtures, sodium acetate, sodium bicarbonate, sodium chloride, sodium citrate, sodium lactate, dibasic sodium phosphate, monobasic sodium phosphate, sodium phosphate mixtures, tromethamine, magnesium hydroxide, aluminum hydroxide, alginic acid, pyrogen-free water, isotonic saline, Ringer's solution, ethyl alcohol, and a combination thereof, but the present invention is not limited thereto.

Exemplary lubricants include magnesium stearate, calcium stearate, stearic acid, silica, talc, malt, glyceryl behenate, hydrogenated vegetable oils, polyethylene glycol, sodium benzoate, sodium acetate, sodium chloride, leucine, magnesium lauryl sulfate, sodium lauryl sulfate, and a combination thereof, but the present invention is not limited thereto.

Exemplary natural oils include almond, apricot kernel, avocado, babassu, bergamot, black current seed, borage, cade, camomile, canola, caraway, carnauba, castor, cinnamon, cocoa butter, coconut, cod liver, coffee, corn, cotton seed, emu, eucalyptus, evening primrose, fish, flaxseed, geraniol, gourd, grape seed, hazel nut, hyssop, isopropyl myristate, jojoba, kukui nut, lavandin, lavender, lemon, litsea cubeba, macadamia nut, mallow, mango seed, meadowfoam seed, mink, nutmeg, olive, orange, orange roughy, palm, palm kernel, peach kernel, peanut, poppy seed, pumpkin seed, rapeseed, rice bran, rosemary, safflower, sandalwood, sasquana, savory, sea buckthorn, sesame, shea butter, silicone, soybean, sunflower, tea tree, thistle, tsubaki, vetiver, walnut, and wheat germ oils, but the present invention is not limited thereto. Exemplary synthetic oils include butyl stearate, caprylic triglyceride, capric triglyceride, cyclomethicone, diethyl sebacate, dimethicone 360, isopropyl myristate, mineral oil, octyldodecanol, oleyl alcohol, silicone oil, and a combination thereof, but the present invention is not limited thereto.

Liquid dosage forms for oral and parenteral administrations include, but are not limited to, pharmaceutically acceptable emulsions, microemulsions, solutions, suspensions, syrups, and elixirs. In addition to the active ingredient, liquid dosage forms may include inert diluents commonly used in the art, for example, water or other solvents, solubilizers, and emulsifiers, such as ethyl alcohol, isopropyl alcohol, ethyl carbonate, ethyl acetate, benzyl alcohol, benzyl benzoate, propylene glycol, 1,3-butylene glycol, dimethylformamide, oil (particularly, cottonseed, peanut oil, corn oil, sprout oil, olive oil, castor oil, or sesame oil), glycerol, tetrahydrofurfuryl alcohol, polyethylene glycol of sorbitan, fatty acid esters, and a mixture thereof. Besides inert diluents, an oral composition may include an adjuvant such as a wetting agent, an emulsifier, a suspending agent, a sweetener, a flavoring agent, or a perfuming agent. In a specific embodiment for parenteral administration, the novel compound of the present invention is mixed with a solubilizer such as Cremophor, alcohol, oil, denatured oil, glycol, polysorbate, cyclodextrin, a polymer, or a combination thereof.

The injectable preparation, for example, a sterile injectable aqueous or oily suspension may be prepared according to the known art using a dispersing or wetting agent, and a suspending agent. A sterile injectable preparation may be a sterile injectable solution, suspension, or emulsion in a parenterally acceptable non-toxic diluent or solvent, for example, a solution in 1,3-butanediol. Those that can be selected among acceptable vehicles and solvents are water, Ringer's solution, U.S.P., and an isotonic sodium chloride solution. In addition, a sterile fixed oil is typically employed as a solvent or suspending medium. To this end, any bland fixed oil containing synthetic mono- or di-glycerides may be employed. In addition, a fatty acid, for example, oleic acid, is used in the preparation of an injection.

An injectable preparation may be sterilized, for example, by filtration through a bacteria-retaining filter, or by adding a sterilizing agent in the form of a sterile solid composition capable of being dissolved or dispersed in sterile water or another sterile injectable medium before use.

To prolong the effect of a drug, it is often preferable to slow the absorption of a drug from subcutaneous or intramuscular injection. This is achieved by using a liquid suspension of a crystalline or amorphous material with low water solubility. The absorption rate of the drug then depends on a dissolution rate, which may ultimately depend on a crystal size and a crystalline form. Alternatively, the delayed absorption of a drug for parenteral administration is achieved by dissolving or suspending the drug in an oil vehicle.

Solid dosage forms for oral administration include capsules, tablets, pills, powders, and granules. In such solid dosage forms, an active ingredient is mixed with one or more inert pharmaceutically acceptable excipients or carriers, such as sodium citrate or calcium diphosphate, and/or a) a filler or extender, such as starch, lactose, sucrose, glucose, mannitol, and silicic acid, b) a binder such as carboxymethylcellulose, alginate, gelatin, polyvinylpyrrolidinone, sucrose, and acacia, c) a humectant such as glycerol, d) a disintegrant such as agar, calcium carbonate, potato or tapioca starch, alginic acid, a specific silicate, and sodium carbonate, e) a dissolution retarding agent such as paraffin, f) an absorption accelerator such as a quaternary ammonium compound, g) a wetting agent such as cetyl alcohol and glycerol monostearate, h) an absorbent such as kaolin or bentonite clay, and i) a lubricant such as talc, calcium stearate, magnesium stearate, solid polyethylene glycol, sodium lauryl sulfate, and a mixture thereof.

The dosage forms such as capsules, tablets, and pills may include a buffer. Similar types of solid compositions may be employed as fillers in soft- and hard-filled gelatin capsules that use lactose, milk sugar, or high molecular weight polyethylene glycol as an excipient. Solid dosage forms such as tablets, dragees, capsules, pills, and granules may be prepared with coatings or shells, such as enteric coatings and other coatings well known in the field of pharmaceutical formulation. These may be compositions that optionally include an opacifying agent, and release only an active ingredient, or preferentially release an active ingredient in specific part of the intestinal tract, optionally, in a delayed manner. Examples of embedding compositions that can be used include polymeric materials and waxes. Similar types of solid compositions may be employed as fillers in soft- and hard-filled gelatin capsules that use lactose, milk sugar, or high molecular weight polyethylene glycol as an excipient.

The active ingredient may be in a micro-encapsulated form with one or more of the excipients described above. Solid dosage forms such as tablets, dragees, capsules, pills, and granules may be prepared with coatings or shells, such as enteric coatings, controlled release coatings, and other coatings well known in the field of pharmaceutical formulation. In these solid dosage forms, the active ingredient may also be mixed with one or more inert diluents such as sucrose, lactose, or starch. These dosage forms may contain additional materials other than inert diluents as in ordinary embodiments, such as tablet lubricants, and other tablet auxiliaries such as magnesium stearate and microcrystalline cellulose. The dosage forms such as a capsule, a tablet, and a pill may include a buffer. These may optionally include an opacifying agent, or compositions that release only an active ingredient, or release an active ingredient preferentially, in a specific part of the intestinal tract, optionally, or in a delayed manner. An example of embedding composition that can be used includes a polymer material and a wax.

Dosage forms for topical and/or transdermal administration of the novel compound of the present invention or a pharmaceutical composition containing the same may include ointments, pastes, creams, lotions, gels, powders, solutions, sprays, inhalants, and/or patches. Generally, the active ingredient is mixed with a pharmaceutically acceptable carrier and/or any necessary preservative and/or a buffer that may be required under sterile conditions. In addition, the present invention considers the use of a transdermal patch, which often has an additional advantage of providing controlled delivery of the active ingredient to the body. Such dosage forms may be prepared, for example, by dissolving and/or dispersing the active ingredient in a suitable medium. Alternatively, or additionally, the rate may be controlled by providing a rate controlling membrane and/or dispersing the active ingredient in a polymer matrix and/or gel.

Preparations for topical administration may include liquid and/or semi-liquid preparations, for example, liniments, lotions, oil-in-water and/or water-in-oil emulsions, such as creams, ointments or pastes, and/or solutions, and/or suspensions, but the present invention is not limited thereto. Although the concentration of the active ingredient may be as high as the solubility limit of the active ingredient in a solvent, a topically administrable preparation may also include, for example, about 1 to 10% (w/w) active ingredient. Preparations for topical administration may further include one or more additional ingredients described herein.

The novel compound of the present invention described herein or a pharmaceutical composition containing the same is typically prepared in a dosage unit form for easy administration and uniform administration. However, it will be understood that the total daily dosage of the composition of the present invention will be determined by an attending physician within the scope of reasonable medical judgment. A specific therapeutically effective dose level for any particular subject will depend on various factors including a disease, a disorder, or a disorder to be treated, and the severity of a disorder; the activity of the specific active ingredient employed; the specific composition employed; the age, weight, overall health, gender, and diet of a subject; the administration time, administration route, and excretion rate of the specific active ingredient employed; the duration of treatment; a drug used in combination or simultaneously with the specific active ingredient employed; and other factors well known in the medical field.

The novel compound of the present invention, a salt thereof, or a pharmaceutical composition thereof may be administered through any route. In some embodiments, the novel compound, a salt thereof, or a pharmaceutical composition thereof is administered via various routes, such as orally, intravenously, intramuscularly, intraarterially, intramedullarily, intrathecally, subcutaneously, intracerebroventricularly, transdermally, intradermally, rectally, intravaginally, intraperitoneally, topically (by a powder, an ointment, a cream, and/or droplets), mucosally, nasally, orally or enterally, sublingually; endotracheal instillation, bronchial instillation, and/or inhalation; and/or by an oral spray, nasal spray, and/or an aerosol. Specifically considered routes are permeable intravenous injection, local administration via blood and/or lymphatic supply, and/or direct administration into an affected area. Generally, the most appropriate route of administration will depend on various factors, including the properties of an agent (e.g., stability in the environment of the gastrointestinal tract), and the discomfort of a subject (e.g., whether a subject can tolerate oral administration).

The exact amount of a compound according to the invention required to achieve an effective amount will vary from subject to subject depending on, for example, the subject's species, age and overall disorder, severity of side effects or disorders, identity of the characteristic compound, mode of administration, etc.

It will be understood that the novel compound and pharmaceutical composition of the present invention can be used in combination therapy. A particular combination of treatments (therapeutic agents or procedures) to be used for combination therapy will determine the desired therapeutic effect to be achieved and the suitability of the desired therapeutic agent and/or procedure.

The pharmaceutical composition of the present invention may be administered alone or in combination with one or more therapeutically active agents. As for "combination," although the following delivery method is within the scope of the present invention, it is not intended to imply that an agent must be administered at the same exact time and/or formulated for delivery together. The composition may be administered simultaneously with, prior to, or subsequent to one or more other desired therapeutic agents or medical procedures. Generally, each agent will be administered at a dose and/or time schedule determined for the agent. In addition, the present invention encompasses delivering a pharmaceutical composition of the present invention in combination with an agent that improves the bioavailability of the composition in the body, reduces and/or modifies its metabolism, inhibits the secretion thereof, and/or modifies the distribution thereof. It will be further understood that the novel compound and therapeutically active agent of the present invention used in this combination may be administered together as a single composition, or separately as different compositions.

For the specific combination used in the combination therapy, a desired therapeutic effect to be achieved and/or procedures including the derivative of the present invention, and/or the suitability of a therapeutically active agent will be considered. It will be understood that used combinations may achieve a desired effect on the same disorder (e.g., the novel compound of the present invention may be administered in combination with another therapeutically active agent used to treat the same disorder), and/or exhibit different effects (e.g., control of any side effects).

"Therapeutically active agent" used herein refers to any material that is used as a medicine to treat, prevent, delay, reduce, or improve a disorder, and a material that is used in treatment, including preventive and curative treatments.

In some aspects, other therapeutically active agents for combination therapy are those for treating a brain disease.

In some embodiments, the pharmaceutical composition of the present invention may be administered in combination with any therapeutically active agent or procedure (e.g., surgery or radiotherapy), which is used to treat, alleviate, improve, or palliate one or more symptoms or characteristics, delay the onset thereof, inhibit the progression thereof, reduce the severity thereof, and/or reduce the occurrence rate.

In one aspect, the present invention may provide a kit for treating a brain disease in a subject suffering from the disease.

In some embodiments, the kit includes i) instructions for administering the novel compound or pharmaceutical composition according to the present invention to a subject suffering from the disease, and ii) the novel compound or pharmaceutical composition according to the present invention. In some embodiments, the kit may include one or more unit dosage forms, which contain the same content of the novel compound or pharmaceutical composition according to the present invention as described herein, effective in treating the disease in a subject. In some embodiments, the subject is a human patient.

In some embodiments, the kit further includes one or more selected from the group consisting of a sterile syringe, a sterile needle, a sterile IV bag, an infusion pump, or any combination thereof.

In yet another aspect, the present invention provides a food composition containing a compound of Chemical Formula 1:

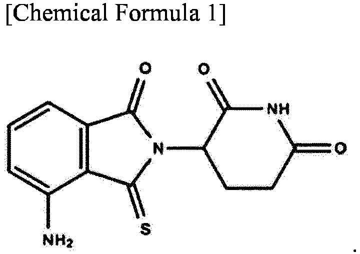

In the present invention, the food may be a health functional food for (i) reducing oxidative stress; (ii) improving motor ability; and/or (iii) improving tyrosine hydroxylase deficiency.

In the present invention, the food may be a heath functional food for aiding the treatment of a brain disease.

The term "food composition" used herein is used in a broad sense to encompass materials containing nutrients, including beverages, tea, drinks, alcoholic beverages, vitamin complexes, prebiotics, probiotics, postbiotics, health supplements, health functional foods, and health foods, and it is used to include not only all foods in a conventional sense, but also a "food additive" or "food additive composition" added to food.

In the present invention, the food composition may be a health functional food having a function of alleviating or improving a brain disease.

The term "health functional food" used herein is the same term as a food for special health use (FoSHU), and refers to a food with high medical and healthcare effects that is processed to efficiently exhibit a bioregulatory function in addition to nutrition supply. Here, "functional" means adjusting nutrients to the structure and function of the human body or obtaining a useful effect for health purposes, such as physiological effects. The food of the present invention may be manufactured by methods commonly used in the art, and may be manufactured by adding raw materials and ingredients commonly added in the art. In addition, the formulation of the food may also be manufactured without limitation as long as it can be a formulation recognized as a food, and the health functional food according to the present invention may be in the form of powder, a granule, a tablet, a capsule, or a beverage.

The health food refers to a food that has an active health maintenance or promotion effect compared to general foods, and "health supplement food" refers to a food for health supplement purposes. In this case, the terms "heath functional food," "health food," and "health supplement food" are used interchangeably.

The food composition may further include a physiologically acceptable carrier, and the type of carrier is not particularly limited, and any carrier commonly used in the technical field may be used.

In addition, the composition may include additional components that are commonly used in food compositions to improve smell, taste, vision, etc. For example, the composition may include vitamin A, C, D, E, B1, B2, B6, or B12, niacin, biotin, folate, or panthotenic acid. In addition, the composition may include a mineral such as zinc (Zn), iron (Fe), calcium (Ca), chromium (Cr), magnesium (Mg), manganese (Mn), copper (Cu), or chromium (Cr). In addition, the composition may include an amino acid such as lysine, tryptophan, cysteine, or valine.

In addition, the composition may include a food additive, for example, a preservative (potassium sorbate, sodium benzoate, salicylic acid, or sodium dehydroacetate), a disinfectant (bleaching powder, advanced bleaching powder, or sodium hydrochlorite), an antioxidant (butylhydroxyanisole (BHA)), or butylhydroxytoluene (BHT)), a coloring agent (tar color), a color fixing agent (sodium nitrite or sodium diacetate), a bleaching agent (sodium sulfite), a seasoning (MSG), a sweetener (dulcin, cyclemate, saccharin, or sodium), a flavoring agent (vanillin or lactone), an expanding agent (alum or D-potassium bitartrate), a strengthening agent, an emulsifier, a thickener (paste), a coating agent, a gum base, an antifoamer, a solvent, or an improving agent. The additive may be selected according to the type of food and may be used at an appropriate amount.

The composition may further include a sitologically acceptable food supplement additive together with the polysaccharide of the present invention, and such an additive may be used appropriately according to a conventional method. The mixing amount of the active ingredient may be appropriately determined depending on the purpose of its use (prevention, health, or therapeutic treatment).

The term "about" used herein may be interpreted to mean approximately, roughly, or to a certain extent. When the term "about" is used with a numerical range, it is interpreted as modifying a corresponding range by extending the boundary above and below a specified numerical value. Generally, the term "about" is used herein to modify a numerical value above and below the specified value by a variance of 10%.

"Individual," "patient," and "subject" are used interchangeably, and encompass any animals including mammals, e.g., mice, rats, other rodents, rabbits, dogs, cats, pigs, cattle, sheep, horses, or primates including humans.

The "treatment" used herein, unless stated otherwise, refers to reversing or palliating the illness or disease to which the term is applied, or one or more symptoms of the illness or disease, or inhibiting the progression thereof, or preventing the illness or disease, and the term "treatment" used herein refers to an action of treating when "treating" is defined as above. Accordingly, "treatment" or "therapy" for a disease in mammals may include one or more the following:
(1) preventing the development of a disease,
(2) preventing the spread of a disease,
(3) reducing a disease,
(4) preventing the relapse of the disease; and
(5) palliating the symptoms of the disease.

"Prevention" used herein refers to, unless otherwise stated, all actions that suppress or delay the onset of an inflammatory disease or angiogenic disease by the administration of the pharmaceutical composition according to the present invention.

The pomalidomide used as a control in the present invention may be represented by (R,S)-4-amino-2-(2,6-dioxopiperidin-3-yl)isoindole-1,3-dione, and represented by Chemical Formula 2.

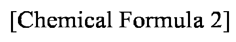

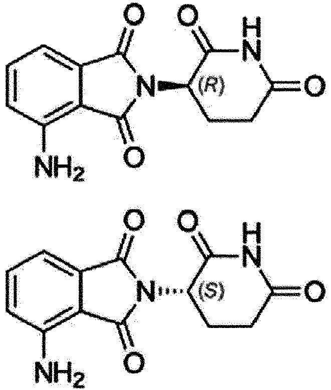

In the present invention, 3,6'-dithiopomalidomide, used as a raw material in synthesis, may be represented by Chemical Formula 3.

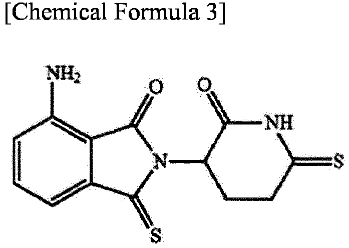

### Examples

Hereinafter, the present invention will be described in more detail with reference to examples. The examples are merely provided to describe the present invention more fully, and it will be obvious to those of ordinary skill in the art that the scope of the present invention is not limited to the following examples.

### Preparation Example

A suspension in which 3,6'-dithiopomalidomide (800 mg, 2.62 mmol, refer to US 9,623,020) was dissolved in a mixed solution of 52 mL of DMSO and 24 mL of H₂O was heated at 100 °C for 17 hours, and then HPLC (254 nm) analysis confirmed 3,6'-DP (0%), 3-MP (76%), and pomalidomide (24%). The dark red solution was cooled to room temperature and diluted with EtOAc. The solution was washed with H₂O twice, and brine once. An aqueous layer was extracted with EtOAc once. Organic layers were combined and dried over MgSO₄, filtered, and then concentrated. The residue was subjected to SiO₂ column chromatography (Loading _ the silica gel obtained by dissolving the residue in a mixture of acetone and the minimum amount of DMSO to coat, mixing it with a silica gel and then rotary-evaporating the silica gel mixture ; Elution _ CHCl₃ : acetone = 20:1 - 9:1) to obtain 3-MP, which was further purified through a CHCl₃ slurry followed by filtration as a dark orange solid (600 mg, 79% F005-02). 3-Thiopomalidomide, which is the compound of Chemical Formula 1, may be abbreviated as 3-MP.

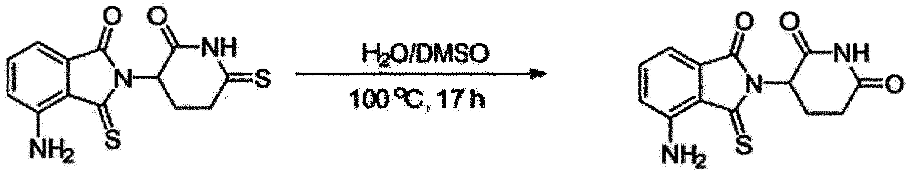

### Example 1. In vitro CRBN binding assay

A thalidomide-based compound is known to regulate immune cell functions and exert various pharmacological effects by binding to cereblon (CRBN), which is an E3 ligase, to degrade transcription factors, Aiolos and Ikaros, as its substrates. Here, the binding affinity of 3-MP to cereblon was compared with that of pomalidomide *in vitro.*

The binding affinity to cereblon was measured using the fluorescence resonance energy transfer (FRET)-based 'AlphaScreen' method, and the corresponding experiment was conducted using a PROTAC Optimization kit for BET bromodomain-cereblon binding kit (#79770, BPS Bioscience, CA USA) according to the 'Competitive Inhibition of the PROTAC assay' described in the manual of the kit. A candidate material, DMSO, and all reagents excluding Flag/Glutathione beads (#6765300, PerkinElmer, USA) included in the kit were used.

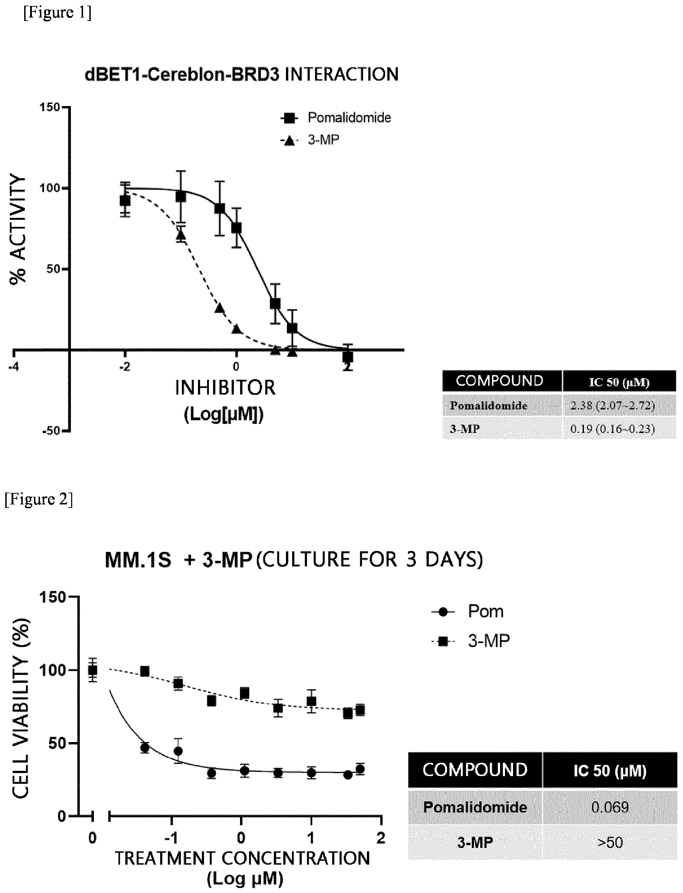

As a result, as shown in FIG. 1, it was confirmed that 3-MP bound more effectively to cereblon than pomalidomide, and the specific IC₅₀ values were 0.19 µM for 3-MP and 2.38 µM for pomalidomide, showing a difference of 10 times or more.

### Example 2. Cytotoxicity evaluation

The MM.1S cell line, which is a human multiple myeloma cell line, was treated with 3-MP and pomalidomide at various concentrations, and cytotoxicity was evaluated.

The MM.1S (CRL-2974) cell line, which is a human multiple myeloma cell line, was purchased from the American Type Culture Collection (ATCC, Manassas, VA, USA) and used, and incubated using RPMI1640 medium (Corning, MD, USA) supplemented with 10% fatal bovine serum (Corning, MD, USA), 100 U/ml of penicillin (Corning, MD, USA), and 100 µg/ml of streptomycin (Corning, MD, USA) at 37 °C in a 5% CO₂ incubator for 24 hours. The cells were seeded at 1 × 10⁴ cells/well in a 96-well plate and cultured for 24 hours, treated with each of pomalidomide and 3-MP at 0, 0.005, 0.014, 0.041, 0.123, 0.37, 1.111, 3.333, or 10 µM, further cultured for 3 days, and then analyzed using a CCK analysis kit (Dojindo, Japan) according to the manufacturer's manual.

As a result, as shown in FIG. 2, the IC₅₀ of pomalidomide was detected to be 0.069 µM, and the IC₅₀ of 3-MP was detected to be 50 µM or more, indicating that 3-MP has no cytotoxicity (blood toxicity) compared to pomalidomide.

### Example 3. Cytotoxicity reduction mechanism

Since it is known that a thalidomide-based compound regulates immune cell functions by binding to the E3 ligase, cereblon (CRBN), to decompose the transcription factors Aiolos and Ikaros as its substrates, it was intended to confirm whether the expression of Aiolos and Ikaros was reduced by 3-MP.

To this end, the MM.1S cell line was cultured in an RPMI medium (Corning, MD, USA) supplemented with 10% fetal bovine serum (Corning, MD, USA), 100 U/ml of penicillin (Corning, MD, USA), and 100 µg/ml of streptomycin (Corning, MD, USA) at 37 °C in a 5% CO₂ incubator. The cells were seeded at 1 × 10⁶ cells/well in a 12-well plate and cultured for 24 hours, and treated with each of pomalidomide and 3-MP at 0.000128, 0.00064, 0.0032, 0.016, 0.08, 0.4, 2, or 10 µM and further cultured for 4 hours. The cells were disrupted by adding a RIPA buffer containing a protease inhibitor cocktail (Thermo Fisher Scientific) and centrifuged at 14,000 rpm for 15 minutes at 4 °C to obtain a cell extract. Equal amounts of the cell extract were loaded, and proteins were separated through SDS-PAGE and then transferred to a PVDF membrane. The protein-transferred membrane was blocked using skim milk, incubated with a primary antibody for 3 hours at room temperature, and then incubated with an HRP-conjugated secondary antibody for 1 hour at room temperature. The resulting product was washed with TBS-T three times between steps. Detection was performed using a chemoluminescence reagent (Thermo Fisher Scientific), and confirmed using Chemidoc (iBright CL1500, Invitrogen, CA, USA). Aiolos (#15103), Ikaros (#9034) and GAPDH (#2118S) antibodies used as primary antibodies and a secondary antibody were purchased from Cell Signaling Technology (Danvers, MA, USA).

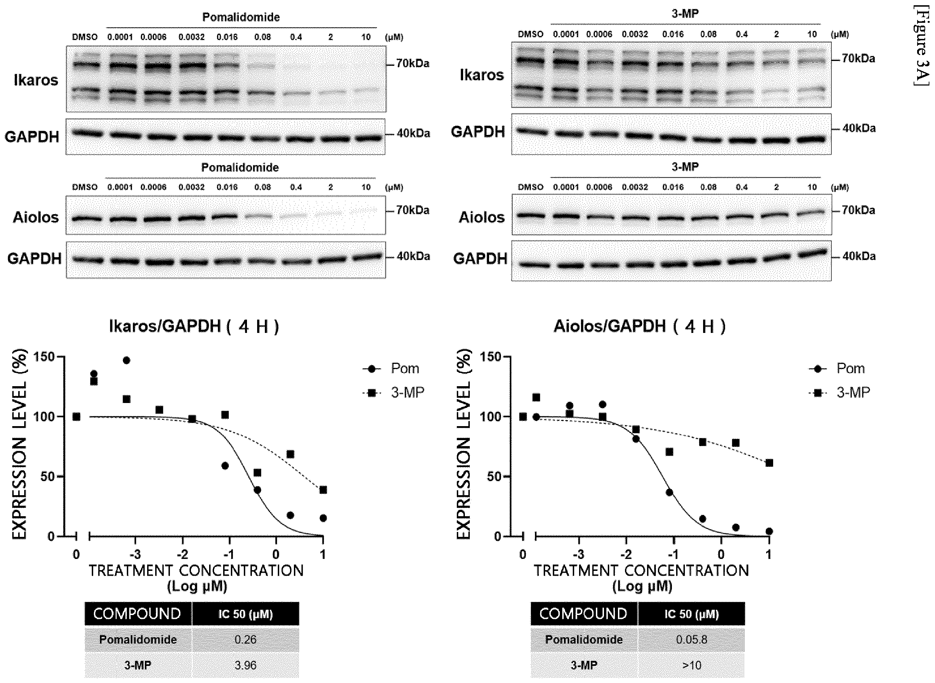

As a result, as shown in FIG. 3A, unlike pomalidomide, it was confirmed that 3-MP does not reduce the expression of Aiolos and Ikaros, which are substrates of cereblon.

Additionally, the expression of Aiolos and Ikaros, mediated by 3-MP, was confirmed over time. After the MM.1S cells were seeded at 1 × 10⁶ cells/well in a 12-well plate and incubated for 24 hours, the cells were treated with each of pomalidomide and 3-MP at 10 µM, further cultured for 1, 4, 8, 12, or 24 hours, disrupted by adding a RIPA buffer containing a protease inhibitor cocktail (Thermo Fisher Scientific), and centrifuged at 14,000 rpm for 15 minutes at 4 °C to obtain a cell extract. Equal amounts of the cell extract were loaded, and proteins were separated by SDS-PAGE and then transferred to a PVDF membrane. The protein-transferred membrane was blocked using skim milk, incubated with a primary antibody for 3 hours at room temperature, and then incubated with an HRP-conjugated secondary antibody for 1 hour at room temperature. The resulting product was washed with TBS-T three times between steps. Detection was performed using a chemoluminescence reagent (Thermo Fisher Scientific), and confirmed using Chemidoc (iBright CL1500, Invitrogen, CA, USA). Aiolos (#15103), Ikaros (#9034) and GAPDH (#2118S) antibodies used as primary antibodies and a secondary antibody were purchased from Cell Signaling Technology (Danvers, MA, USA).

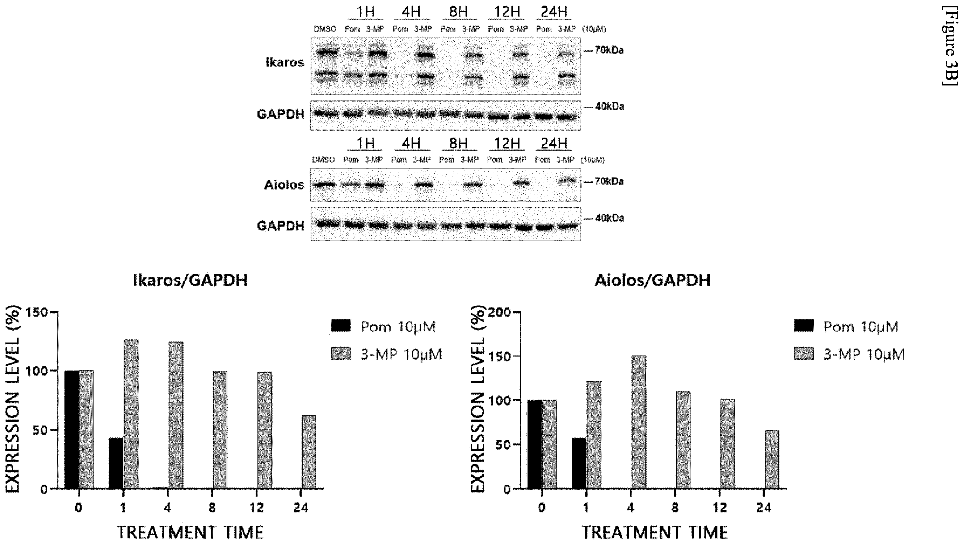

As a result, as shown in FIG. 3B, it was confirmed that 3-MP does not reduce the expression of Aiolos and Ikaros, which are substrates of cereblon, compared to pomalidomide.

### Example 4. Reproductive toxicity reduction mechanism

It has been reported that a cereblon (CRBN)-binding therapeutic such as a thalidomide-based compound can cause severe congenital abnormalities such as forelimb shortening or brachydactyly, which are closely associated with the decomposition of SALL4. Accordingly, the SALL4 decomposition effect of 3-MP was compared with that of pomalidomide.

To this end, the Tera-1 cell line (HTB-105), which is a human embryonic carcinoma cell line, was purchased from the American Type Culture Collection (ATCC, Manassas, VA, USA) and used, and then cultured using a DMEM medium (Corning, MD, USA) supplemented with 10% fetal bovine serum (Corning, MD, USA), 100 U/ml of penicillin (Corning, MD, USA), and 100 µg/ml streptomycin (Corning, MD, USA) at 37 °C in a 5% CO₂ incubator.

The cells were seeded at 5 × 10⁵ cells/well in a 12-well plate, cultured for 24 hours, and then treated with each of pomalidomide or 3-MP at 0.000128, 0.00064, 0.0032, 0.016, 0.08, 0.4, 2, or 10 µM and further cultured for 4 hours. The cells were disrupted by adding a RIPA buffer containing a protease inhibitor cocktail (Thermo Fisher Scientific), and centrifuged at 14,000 rpm for 15 minutes at 4 °C to obtain a cell extract. Equal amounts of the cell extract were loaded, and proteins were separated by SDS-PAGE and then transferred to a PVDF membrane. The protein-transferred membrane was blocked using skim milk, incubated with a primary antibody for 3 hours at room temperature, and then incubated with an HRP-conjugated secondary antibody for 1 hour at room temperature. The resulting product was washed with TBS-T three times between steps. Detection was performed using a chemoluminescence reagent (Thermo Fisher Scientific), and confirmed using Chemidoc (iBright CL1500, Invitrogen, CA, USA). Aiolos (#15103), Ikaros (#9034) and GAPDH (#2118S) antibodies used as primary antibodies and a secondary antibody were purchased from Cell Signaling Technology (Danvers, MA, USA).

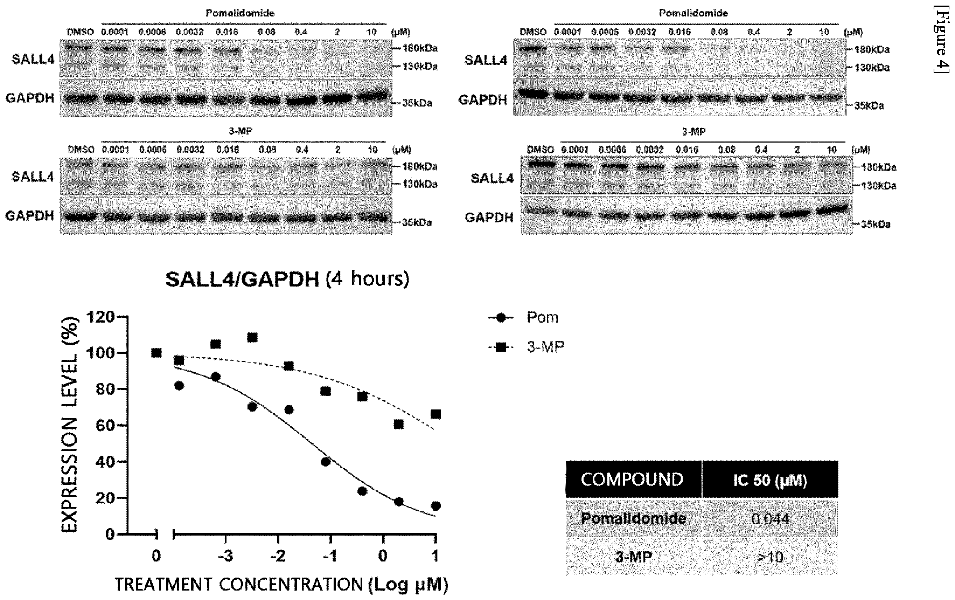

As a result, as shown in FIG. 4, it was confirmed that 3-MP shows less decomposition of SALL4 than pomalidomide, suggesting that 3-MP can suppress teratogenic side effects better than pomalidomide.

### Example 5. Oxidative stress suppression effect

To confirm an oxidative stress suppression effect, the expression levels of iNOS and COX-2 were confirmed.

To this end, the RAW264.7 cell line, which is a mouse macrophage cell line, was purchased from the Korea Cell Line Bank (KCLB, Korea) and used, and cultured in a DMEM medium (Corning, MD, USA) supplemented with 10% fetal bovine serum (Corning, MD, USA), 100 U/ml of penicillin (Corning, MD, USA), and 100 µg/ml of streptomycin (Corning, MD, USA) at 37 °C in a 5% CO₂ incubator.

The RAW264.7 cells were seeded at 2.4 × 10⁵ cells/well in a 12-well plate and cultured for 24 hours, and then pretreated with each of pomalidomide and 3-MP at 3.33, 10, or 30 µM. After 1 hour, the cells were stimulated by treatment with 60 ng/ml of LPS, and further cultured for 24 hours. The cells were disrupted by adding a RIPA buffer containing a protease inhibitor cocktail (Thermo Fisher Scientific) and centrifuged at 14,000 rpm for 15 minutes at 4 °C to obtain a cell extract. Equal amounts of the cell extract were loaded, and proteins were separated through SDS-PAGE and then transferred to a PVDF film. The protein-transferred membrane was blocked using skim milk, incubated with a primary antibody for 3 hours at room temperature, and then incubated with an HRP-conjugated secondary antibody for 1 hour at room temperature. The resulting product was washed with TBS-T three times between steps. Detection was performed using a chemoluminescence reagent (Thermo Fisher Scientific), and confirmed using Chemidoc (iBright CL1500, Invitrogen, CA, USA). iNOS (#13120) COX-2 (#12282) and GAPDH (#2118) antibodies used as primary antibodies and a secondary antibody were purchased from Cell Signaling Technology (Danvers, MA, USA).

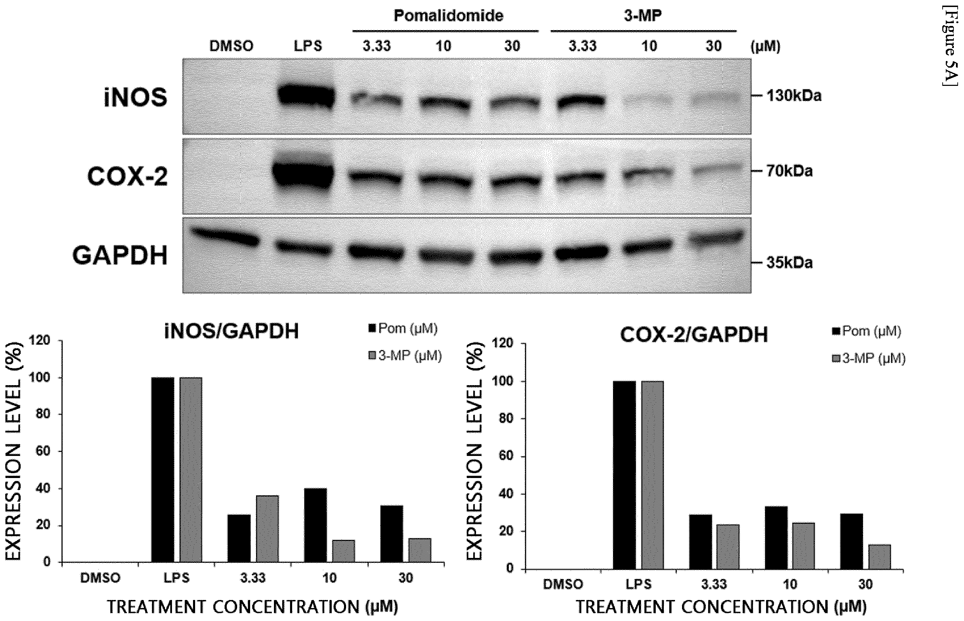

As a result, as shown in FIG. 5A, it can be seen that 3-MP inhibit the expression of iNOS and COX-2 to effectively inhibit oxidative stress, and exhibited a better oxidative stress suppression effect than pomalidomide.

To further confirm the oxidative stress suppression effect, the content of nitrite released due to an inflammatory response and the expression levels of iNOS, which is an enzyme that produces nitric oxide, and COX-2, which is cyclooxygenase-2, were determined in mouse microglial cells (BV-2, ATCC, Manassas, VA, USA).

To determine the content of nitrite and the protein expression levels of iNOS and COX-2, BV-2 was seeded in a 12-well plate (Corning, MD, USA) at 1.5 × 10⁵ cells/well, and cultured using a DMEM medium (Corning, MD, USA) containing 10% fetal bovine serum (Coming, MD, USA) and 100 U/ml of penicillin/streptomycin (Corning, MD, USA) at 37 °C in a 5% CO₂ incubator for one day for stabilization. A 10 mM DMSO stock (Sigma, USA) of pomalidomide and 3-MP was pretreated with the same medium as the culture solution of BV-2 at 30, 10, or 3.33 µM for 1 hour. 1 µg/mL of LPS (Sigma, USA) was added to all experimental groups excluding the non-inflammation-induced group and allowed to react at 37 °C in a 5% CO₂ incubator for 24 hours.

The cell culture solution was collected to measure the content of nitrite, and centrifuged at 3,000 rpm for 5 minutes to obtain a supernatant. The concentration of nitrite in the cell culture solution was measured using a microplate reader (NEO2MALPHAB Synergy Neo-2, BioTek, CA, US) according to the manual of the Griess Reagent System (#G2930, Promega, WI, USA).

The cells from which the cell culture solution was harvested were disrupted by adding a RIPA buffer containing a protease inhibitor cocktail (Thermo Fisher Scientific), and centrifuged at 14,000 rpm for 15 minutes at 4 °C to obtain a cell extract. Equal amounts of the cell extract were loaded, and proteins were separated through SDS-PAGE and then transferred to a PVDF membrane. The protein-transferred membrane was blocked using skim milk, incubated with a primary antibody for 3 hours at room temperature, and then incubated with an HRP-conjugated secondary antibody for 1 hour at room temperature. The resulting product was washed with TBS-T three times between steps. Detection was performed using a chemoluminescence reagent (Thermo Fisher Scientific), and confirmed using Chemidoc (iBright CL1500, Invitrogen, CA, USA). iNOS (#13120) COX-2 (#12282) and GAPDH (#2118), which are used as primary antibodies, and secondary antibodies corresponding thereto were purchased from the Cell Signaling Technology (Danvers, MA, USA).

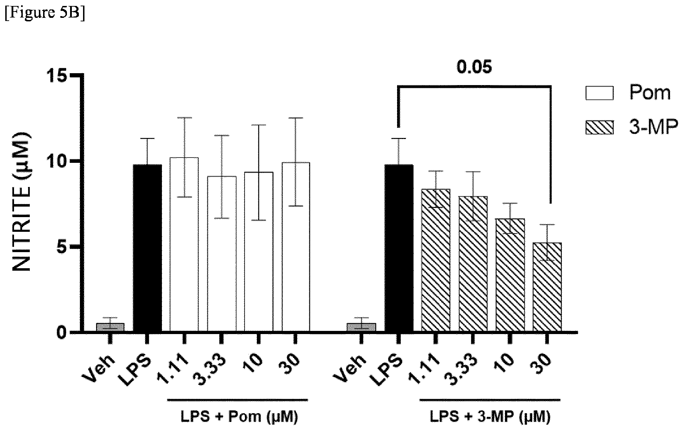

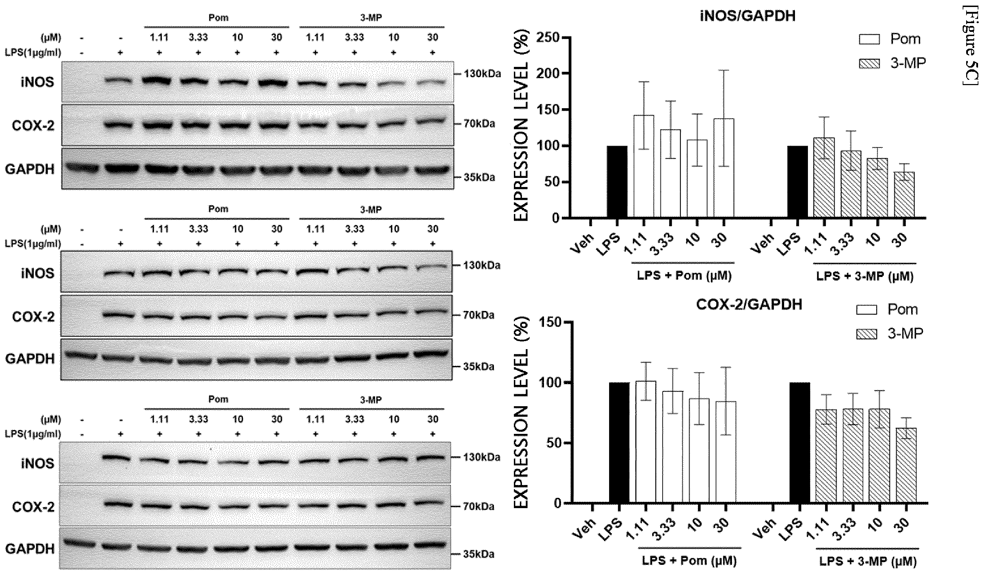

As a result, as shown in FIG. 5B, 3-MP effectively reduced the amount of the residual nitrite released by LPS, compared to pomalidomide, and as shown in FIG. 5C, the expression of the oxidative stress-associated proteins (iNOS and COX-2) was also effectively reduced. Thus, it was confirmed that 3-MP exhibited a much better oxidative stress suppression effect than pomalidomide.

Finally, to determine the mRNA expression levels of iNOS and COX-2, BV-2 was seeded in a 6-well plate (Corning, MD, USA) at 5x10⁵ cells/well, and cultured using a DMEM medium (Corning, MD, USA) containing 10% fetal bovine serum (Corning, MD, USA) and 100 U/ml of penicillin/streptomycin (Corning, MD, USA) at 37 °C in a 5% CO₂ incubator for one day for stabilization. A 10 mM DMSO stock (Sigma, USA) of pomalidomide and 3-MP was pretreated with the same medium as in the culture solution of BV-2 at 30, 10, or 3.33 µM for 1 hour. 1 µg/mL of LPS (Sigma, USA) was added to all experimental groups excluding the non-inflammation-induced group and allowed to react at 37 °C in a 5% CO₂ incubator for 6 hours.

The drug-treated BV-2 underwent RNA prep according to the kit manual using an RNA prep kit (Monarch Total RNA Miniprep kit, NEB, UK), and the concentration was measured using a nanophotometer (NP80, Implen, Germany). According to the concentration of each RNA sample, 1 µg of RNA was added and cDNA was synthesized according to the manual of the cDNA synthesis kit (LunaScript RT SuperMix kit, NEB, UK).

The expression level of each oxidative stress-associated gene in the synthesized cDNA was analyzed and compared using Real-Time PCR (StepOne^{™} Bioscience, USA) equipment. For analysis, in each well of a 384-well plate (PCR Plate, 384-well, Thermo Fisher, USA), 1 µg of cDNA, and 1µg each of 10 pmol forward and reverse primers per gene, 5 µl of D.W., and 10 µl of SYBR green (Luna^{®} Master Mix, NEB, UK) were included. PCR was performed according to the manual of the SYBR green kit, and the base sequences of the used primers are shown in Table 1. The C_{T} value for the gene of each sample obtained through real-time PCR was normalized by the value of the internal control gene (GAPDH) of each sample, the expression level was estimated inversely by 2ΔΔC_{T} calculation, and the resulting value of each group was quantified and expressed as a percentage relative to the LPS- only treatment group.

**[Table 1]**

| SEQ ID NO: | Name of primer | Sequence (5' →3') |
|---|---|---|
| 1 | Mouse iNOS Forward | F: GCTATGGCCGCTTTGATGTG |
| 2 | Mouse iNOS Reverse | R: TTGGGATGCTCCATGGTCAC |
| 3 | Mouse COX-2 Forward | F:CTGGAACATGGACTCACTCAGTTTG |
| 4 | Mouse COX-2 Reverse | R: AGGCCTTTGCCACTGCTTGT |
| 5 | Mouse GAPDH Forward | F:TTCAACAGCAACTCCCACTCTTCC |
| 6 | Mouse GAPDH Reverse | R: TGGTCCAGGGTTTCTTACTCCTT |

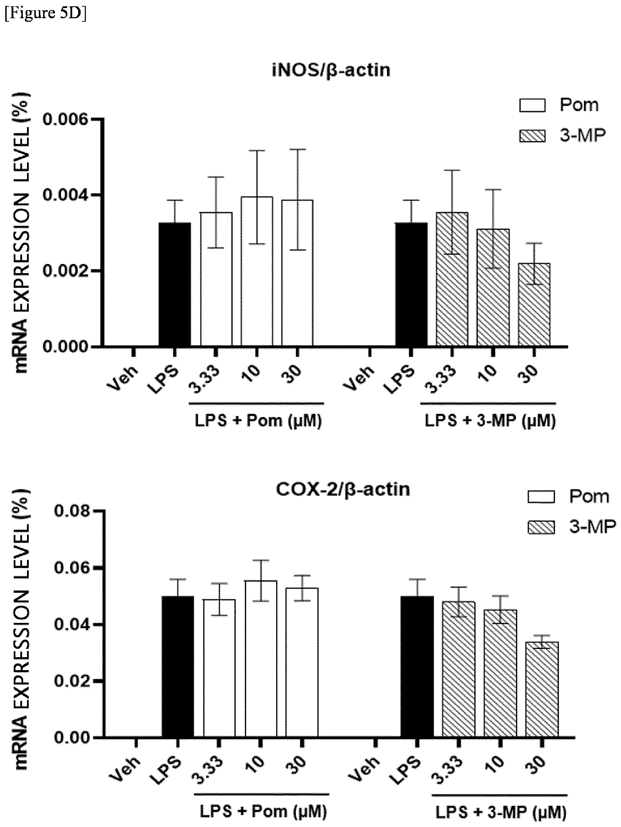

As a result, as shown in FIG. 5D, it was confirmed that 3-MP can effectively inhibit the mRNA expression of an oxidative stress-associated gene, and exhibit a better oxidative stress suppression effect than pomalidomide.

### Example 6. Verification of in vivo 3-MP stability

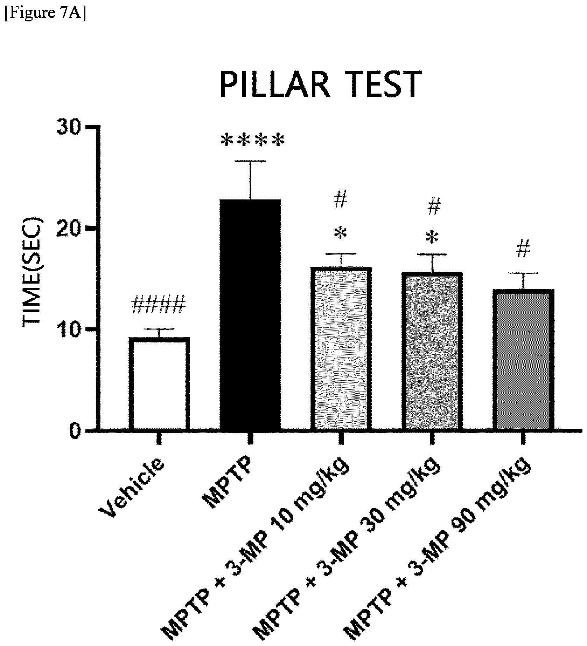

A stock sample was prepared by dissolving the 3-MP standard material in dimethyl sulfoxide (DMSO) to 1 mg/mL. To plot a standard calibration curve, the stock sample was diluted by half with acetonitrile from 125 to 0.39 µg/mL to prepare standard solutions for a total of 10 samples at different concentrations, and the calibration curve was plotted as shown in FIG. 7A.

For a test drug to verify stability in plasma, an experiment was conducted by diluting 3-MP to a concentration of 100 µg/mL. For the experiment of verifying stability in plasma, 10 µL of the test drug prepared at a concentration of 100 µg/mL was added to 90 µL of human plasma and mixed for 30 seconds, and then immediately after the addition, the plasma-mixed test drug was incubated for 15, 30, 60, or 120 minutes to prepare samples. Afterward, 400 µL of 100% ethyl acetate was added to each sample and extracted using a vortex mixture for 1 minute. The extracted sample was centrifuged at 4 °C and 13,000 rpm for 10 minutes, and a supernatant was collected and then transferred to a new vial. A plasma extraction sample was injected into a UPLC-LTQ-Orbitrap 240 (Thermo Fisher Scientific, USA)-coupled (coupled in-line) Hypersil GOLDTM C18 column (2.1 × 100 mm, 1.7 µm; Thermo Scientific, USA). The column temperature was 25 °C, mobile phase A was a mixed solution of water and formic acid (100:0.1, v/v), and mobile phase B was a mixed solution of methanol and formic acid (100:0.1, v/v). Initially, the mobile phase B was increased from 5% to 95% until 9 minutes and analyzed, the mobile phase B was maintained 95% until 12 minutes, and then the mobile phase B was lowered to 5% and equilibrated, and used in the subsequent analysis, the flow rate of the mobile phase was 0.3 mL/min, the sample injection volume was 2 µL, and the mass spectrometer was operated in the ESI-positive mode. The spray voltage was set to 3.5 kV, and the flow rates of the nitrogen sheath gas, auxiliary gas, and sweep gas were analyzed as 50, 10, and 1 (arbitrary units), respectively. The capillary temperature was maintained at 250 °C. Orbitrap data was collected in the range of m/z 100-1,000, and analytical data was analyzed using Excalibur 4.0 software (Thermo Fisher Scientific, USA).

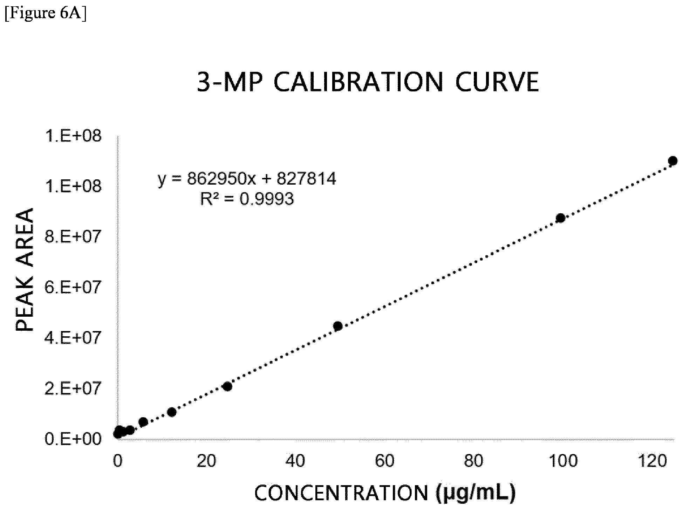

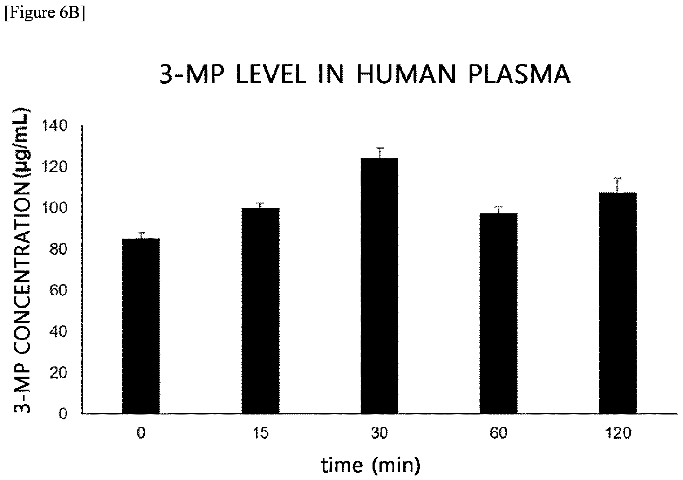

As a result of confirming the 3-MP level in human plasma through quantification based on the calibration curve of FIG. 6A, as shown in FIG. 6B, 3-MP was confirmed to have a plasma level of 80 µg/mL immediately after plasma addition, that is, at 0 minutes, and showed a somewhat increasing and decreasing pattern over time. However, it was confirmed that 3-MP could be maintained without significant changes in plasma from 0 to 120 minutes.

### Example 7. Therapeutic effect of 3-MP on Parkinson's disease

### 7-1. Induction of disease model and drug administration

1-Methyl-4-phenyl-1,2,3,6-tetrahydropyridine (MPTP) is a precursor of 1-methyl-4-phenylpryridium (MPP+), which is a neurotoxin known to cause permanent Parkinson's disease by destroying dopaminergic nerve tissue located in the substantia nigra of the brain.

To confirm the efficacy of 3-MP in a Parkinson's disease model by MPTP administration, sixty 6-week-old male C57BL/6 mice were ordered (Koatech, Korea) and divided into 5 groups according to the administered drug after a one-week acclimatization period (12 per group): control (G1), MPTP-administered group (G2), MPTP + 3-MP (10 mg/kg/day) group (G3), MPTP + 3-MP (30 mg/kg/day) group (G4), MPTP + 3-MP (90 mg/kg/day) group (G5).

To cause the disease, mice in the G2, G3, G4, and G5 groups were administered MPTP dissolved in saline via an intraperitoneal route at 2-hour intervals four times a day (20 mg/kg/time), while the G1 group received the same amount of saline. Afterward, the mice in the G3, G4, and G5 groups were administered 3-MP suspended in 10 ml/kg of 1% sodium carboxymethylcellulose (Sigma, C4888, in Distilled water) once a day via an oral route for a total of 7 times, and the G1 and G2 groups received the same amount of 1% sodium carboxymethylcellulose.

### 7-2. Behavioral assessment (pillar test)

Using a wooden pillar (length: 80 cm, diameter: 0.8-1 cm) placed vertically on the cage floor, mice were trained to turn around from the top of the pillar in the opposite direction and descend to the bottom three times a day on days 2 - 3 of the test. On day 4 of the test, the same test was performed three times, and the time it took to change direction from the top and descend.

As a result, as shown in FIG. 7A, it was confirmed that the increased column descent time by MPTP administration was reduced to a statistically significant level by the administration of all doses of 3-MP, and as the administration concentration increased, an overall tendency for the descent time to decrease as the dose concentration increases was observed. In the highest dose administration group, which is the 90 mg/kg administration group, it was confirmed that the descent time was recovered to a level similar to that of the vehicle administration group.

### 7-3. Behavioral assessment (grip strength test)

A grip strength test was performed on day 6 of the test. When a mouse put on a grip strength measuring device (SLD-5FGN, Duratool, China) pulled a net of the device, the measuring device automatically measured the animal's grip strength and expressed it in grams. The average value was measured through five tests per individual.

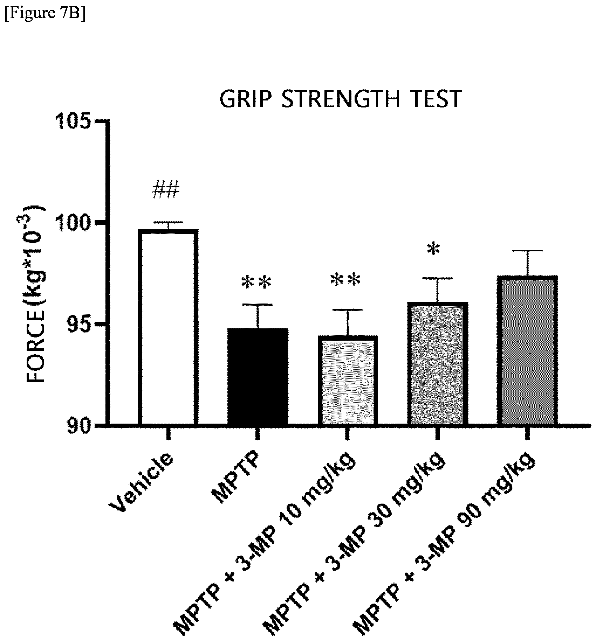

As a result, as shown in FIG. 7B, it was confirmed that, compared to the MPTP administration group, in the 3-MP administration group, the grip strength increased in a concentration-dependent manner, and in the highest dose administration group, which is the 90 mg/kg administration group, the grip strength level is recovered to a level similar to that of the vehicle administration group.

### 7-4. Immunohistochemical test

After the end of the test on day 7, the brain tissue of the sacrificed mice was collected, sectioned, and analyzed.

Six mice per group were fixed by perfusion with saline followed by perfusion with 4% paraformaldehyde (Biosesang, cat#PC2031-000-00). Each brain tissue was rapidly frozen after isolation and stored at -80 °C until tissue sectioning. Sections for immunohistochemical testing were embedded in an OCT compound (SAKURA, cat#4583), samples were sectioned into 20 µm-thick sections using a cryomicrotome, Leica CM1950 (Leica Microsystems, Germany), and each section was stored at 4 °C until an antibody reaction. The striatum and substantia nigra of the brain were placed on a microscope slide, each slide was washed with PBS (Biosesang, cat#PR2007-000-00) containing 0.3% Triton X-100 (Sigma Aldrich, cat#X100), and peroxidase blocking was performed at room temperature for 15 minutes. Each slide was washed three times with PBS containing 0.3% Triton X-100, and blocked with a solution of 5% normal goat serum (Sigma Aldrich, cat#S26) in PBS containing 0.3% Triton X-100 at room temperature for one hour. A tyrosine hydroxylase antibody (abeam, ab112) was diluted 1:1000 and applied to the slides overnight at 4 °C. The slide was washed with PBS and allowed to react with a secondary antibody (DAKO, cat#K5007) at room temperature for 1 hour. It was washed with PBS, treated with DAB (DAKO, cat#K5007), and after a 2-3-minute reaction, observed using a microscope. The DAB reaction was stopped by washing with PBS, dehydrated with xylene, and covered with a cover slide. Images stained with the tyrosine hydroxylase antibody were observed under a microscope (Olympus, SZ61, BX51), and the OD values, which are immunostaining-positive, observed in the striatum and substantia nigra tissue were measured and analyzed by the Image J program.

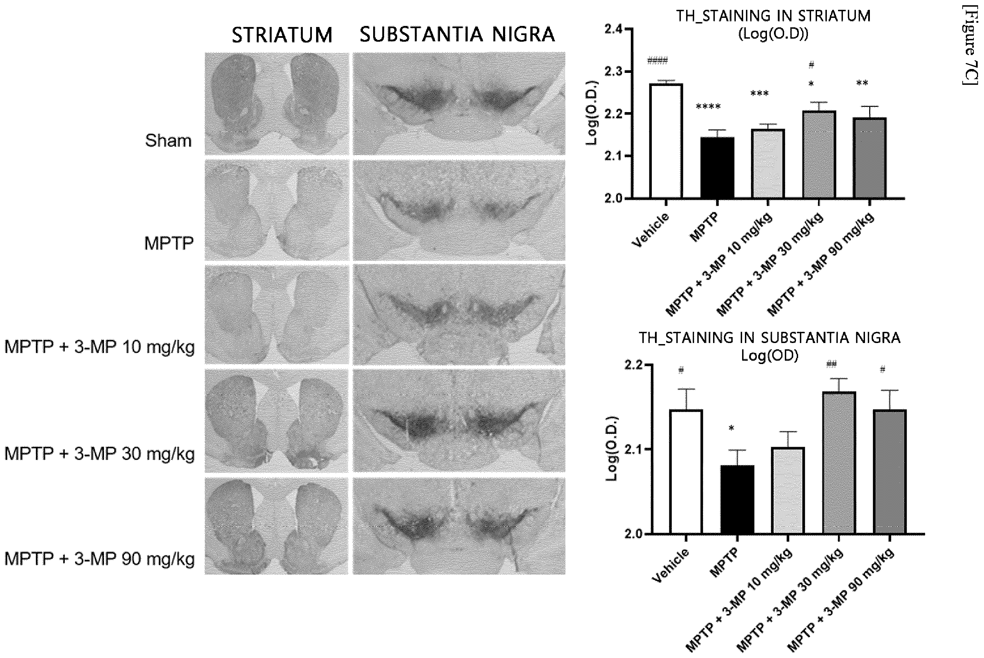

As a result, as shown in FIG. 7C, the expression of tyrosine hydroxylase (TH) in the striatum was significantly increased in all 3-MP administration groups, particularly, the 3-MP 30 mg/kg administration group, compared to the MPTP administration group. The expression of tyrosine hydroxylase in the substantia nigra was increased in a statistically significant manner in the 3-MP 30 and 90 mg/kg administration groups, compared to the MPTP administration group.

As above, as specific parts of the present invention have been described in detail, although it is clear to those skilled in the art that this these specific parts are merely preferred embodiments, and the scope of the present invention is not limited thereto. Thus, the substantial actual scope of the present invention will be defined by the accompanying claims and their equivalents.

## Claims

1. A pharmaceutical composition for preventing or treating a brain disease, comprising a compound of Chemical Formula 1 or a pharmaceutically acceptable salt thereof as an active ingredient:

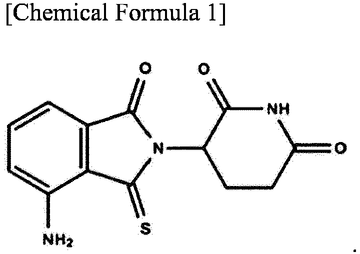

2. The pharmaceutical composition of claim 1, wherein the brain disease is a brain disease caused by oxidative stress.

3. The pharmaceutical composition of claim 1, wherein the brain disease is selected from the group consisting of a traumatic brain injury, Alzheimer's disease, Parkinson's disease, Huntington's disease, Lou Gehrig's disease, multiple system atrophy, Alzheimer's dementia, vascular dementia, frontotemporal dementia, Lewy dementia, ischemic stroke, hemorrhagic stroke, and multiple sclerosis.

4. The pharmaceutical composition of claim 1, wherein the compound of Chemical Formula 1 is non-teratogenic.

5. The pharmaceutical composition of claim 1, wherein the pharmaceutical composition is orally or parenterally administered.

6. The pharmaceutical composition of claim 1, wherein the compound of Chemical Formula 1 or a pharmaceutically acceptable salt thereof
(i) improves motor ability; and/or
(ii) increases the expression of tyrosine hydroxylase (TH) in the striatum and/or substantia nigra of the brain.

7. A food composition comprising a compound of Chemical Formula 1:

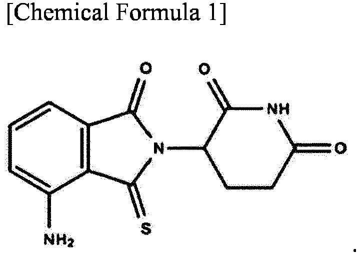

8. The food composition of claim 7, wherein the food is a health functional food for (i) reducing oxidative stress; (ii) improving motor ability; and/or (iii) improving tyrosine hydroxylase deficiency.

9. The food composition of claim 7, wherein the food is a health functional food for aiding the treatment of a brain disease.
